# EUROPEAN PATENT SPECIFICATION

(11) **EP 3 196 307 B1**
(45) Date of publication and mention of the grant of the patent: **26.12.2018**
(21) Application number: 15888619.2
(22) Date of filing: 27.08.2015
(51) Int. Cl.: A61P 21/00, A61P 25/02, A61K 48/00, A61K 38/18

(54) **CODON-OPTIMIZED RECOMBINANT PLASMID, CONTAINING VEGF AND FGF2-CODING GENES, FOR USE IN PERIPHERAL NERVE REGENERATION**
CODON-OPTIMIERTES REKOMBINANTES PLASMID, ENTHALTEND VEGF- UND FGF2-CODIERENDE GENE, ZUR VERWENDUNG IN DER REGENERATION VON PERIPHEREN NERVEN
PLASMIDE RECOMBINANT OPTIMISÉ PAR CODON, CONTENANT DES GÈNES CODANTS VEGF ET FGF2, POUR L'UTILISATION DANS LA RÉGÉNÉRATION DES NERFS PÉRIPHÉRIQUES.

(30) Priority: 16.09.2014 RU 2014137218
(43) Date of publication of application: 26.07.2017
(73) Proprietor: "Nextgen" Company Limited, Moscow 119333 (RU)
(72) Inventor: ISAEV, Artur Aleksandrovich, Moscow 119607 (RU); RIZVANOV, Albert Anatolyevich, Kazan 420127 Republic of Tatarstan (RU); MASGUTOV, Ruslan Faridovich, Kazan 420049 Republic of Tatarstan (RU); BOGOV, Aleksei Andreevich, Kazan 420097 Republic of Tatarstan (RU); SALAFUTDINOV, Ilnur Ildusovich, Kazan 420101 Republic of Tatarstan (RU); DEEV, Roman Vadimovich, St.Petersburg 192029 (RU); BOZO, Ilya Yadigerovich, Kuvshinovo Tverskaya Oblast 172110 (RU); PLAKSA, Igor Leonidovich, Vyborgsky r-n Leningradskaya Oblast 188855 (RU); BOGOV, Andrei Alekseevich, Kazan 420097 Republic of Tatarstan (RU)
(74) Representative: Glawe, Delfs, Moll
(86) International application number: PCT/RU2015/000545
(87) International publication number: WO 2016/163912

(56) References cited:
- EP-A2- 1 732 614
- RU-C1- 2 459 630
- RU-C2- 2 517 117
- S. I. NIKOLAEV ET AL: "Poly([epsilon]-Caprolactone) Nerve Conduit and Local Delivery of vegf and fgf2 Genes Stimulate Neuroregeneration", BULLETIN OF EXPERIMENTAL BIOLOGY AND MEDICINE, vol. 157, no. 1, 1 May 2014 (2014-05-01), pages 155-158, XP055454415, US ISSN: 0007-4888, DOI: 10.1007/s10517-014-2513-1
- ORLI THAU-ZUCHMAN ET AL: "Combination of Vascular Endothelial and Fibroblast Growth Factor 2 for Induction of Neurogenesis and Angiogenesis after Traumatic Brain Injury", JOURNAL OF MOLECULAR NEUROSCIENCE, HUMANA PRESS INC, NEW YORK, vol. 47, no. 1, 13 January 2012 (2012-01-13), pages 166-172, XP035041248, ISSN: 1559-1166, DOI: 10.1007/S12031-012-9706-8
- MASGUTOV R.F.: 'Stimuliatsiia posttravmaticheskoi regeneratsii sedalishchnogo nerva krysy s pomoshchiu plazmidy, ekspressirushchei sosudistyi endotelialnyi faktor rosta i osnovnoi faktor rosta fibroblastov' 2011, pages 67 - 70, XP009502189

## Description

### The Prior State of technique

According to different data the incidence of peripheral nerve injuries of various etiology is presented in 3-10% of the population [1-3]. The rationale to develop additional methods of treatment which can improve the quality of standard ones is due to a prolonged period of rehabilitation (a year and longer), a significant decrease of a life quality of working-age patients and a high incapacitation rate. A peripheral nerve injury is a common cause of occupational disability. The selection of a method of peripheral nerve integrity repair is due to a number of peculiarities of an injury in every individual case, namely a mechanism of injury, the time elapsed from an injury sustained to a surgical intervention, the extent of a peripheral nerve defect and so on. A suture of incised nerve regions though an end-to-end anastomosis is one of reconstructive treatment modalities. However, a peripheral nerve injury is often accompanied with a critical defect, thereby rendering this approach inapplicable. In this setting, autologous nerve grafting is the most appropriate option to repair the nerve integrity. A functionally less significant nerve can be harvested as an autologous graft. Different conduits can be used as an alternative. These are tubular structures which are designated to replace an extended tissue defect and to create conditions for peripheral nerve regeneration.

The degree of extremity function recovery innervated by a peripheral nerve after surgical repair depends upon a variety of factors such as the time elapsed since an injury sustained to surgery, a defect extent, the distance from the injury location of the peripheral nerve to the area innervated and so on. However, despite the advances in the technique of a nerve integrity reconstruction only a partial recovery of the function of the extremity innervated occurs as a rule even under the most favorable conditions. This induces the search for new methods of treatment which would improve the results of a standard reconstructive treatment and the quality of patients' life in general.

The use of growth factors to induce the regeneration of a peripheral nerve is one of these approaches. This concept has resulted from the accumulation of knowledge on a significant role growth factors play in the natural process of peripheral nerve regeneration [4].

A vascular endothelial growth factor (VEGF) is one of the well-studied growth factors affecting the recovery of peripheral nerves. VEGF is one of the main regulators of angiogenesis and vasculogenesis. VEGF is a dimer 34-42 kDa disulfide-bound glycoprotein. VEGF-A is a specific mitogen for endothelial cells (ECs). VEGF induces the proliferation of ECs, their activation, differentiation and their forming capillary tubules, further remodelling into mature blood vessels. It is also a strong factor increasing the EC survival as it induces the expression of antiapoptotic proteins. Deletions of the genes coding VEGF result in serious defects in the process of the cardiovascular system development that is fatal.

A gene of human VEGF is located in a chromosomal locus 6p21.3. The coding region comprises about 14,000 bps. There are several VEGF isoforms such as VEGF 121, VEGF 145, VEGF 148, VEGF 165, VEGF 183, VEGF 189, VEGF 206, resulting from alternative splicing of mRNA which consists of 8 exons. A particular extracellular localization corresponds to each VEGF isoform, which is based on their biochemical differences in the ability to bind heparin- and heparan-sulphate. For example, all transcripts of the human VEGF-A gene contain the exons 1-5 and 8, the differences are due to alternative splicing of the exons 6 and 7.

After the discovery VEGF has long been considered only as an inductor of angiogenesis and a potential therapeutic agent for the treatment of different disorders accompanied with tissue ischemia. However, over the time the data on its neuroprotective properties for neurons of both the peripheral nervous system and the central one have been obtained [5, 6]. VEGF stimulates the proliferation of Schwann cells, astrocytes, microglia and cortical neurons [7-10]. A significant increase of the expression of VEGF and Flt-1 (VEGF type II receptor) in the lumbar spine in response to injury was shown in a rat sciatic nerve crush injury model [11]. Thus, the prerequisites for using this growth factor as an additional therapeutic component of reconstructive treatment of peripheral nerve injuries have originated. The use of VEGF as part of matrigel filling in a conduit induces axonal sprouting that manifests as an increased axon number in the conduit per a unit of the cross section area [12].

The use of VEGF-loaded poly-lactic acid microspheres in an autologous vein graft in a model of trauma with an extensive defect of fibular and tibial nerves significantly improves the nerve functional index and increases the number of myelinated fibers in the graft [13]. In an experiment VEGF induces the Schwann cells division and migration in a graft towards the distal parts that correlates with an increased number of capillaries and myelinated fibers [14]. The introduction of VEGF in combination with BDNF into cavernosal bodies in a rat cavernous nerve injury model resulted in the recovery of the lost innervation and erectile function [15].

FGF is another growth factor which has the properties to induce neurogenesis. FGF induces the Schwann cell proliferation and migration in a peripheral nerve injury [16]. In an animal experiment it was shown that the blocking of receptors to FGF, Fgfr1 and Fgfr2, caused neuropathy of non-myelinating sensory fibers and a significant impairment of thermal pain sensitivity [17]. The bone marrow-derived stem cells application in a peripheral nerve injury model resulted in the increased FGF expression that, by the authors' opinion, induced the Schwann cell migration and proliferation [18]. In a thoracic spinal cord injury model the use of FGF in a sciatic nerve graft promoted the improvement of the upper extremity motor function [19]. Thus, based on the experimental data it can be supposed that these growth factors incorporation in complex therapy of a peripheral nerve repair can be effective.

However, the therapeutic application of growth factors is known to have a number of limitations. After administration into an injury site they undergo a rapid degradation therefore their constant concentration cannot be maintained to achieve the desired therapeutic effect [20]. Thereby, the use of gene therapy is much more suitable. There are two main trends such as the use of viral and non-viral vectors based on the mechanism of transfer of a gene encoding a therapeutic agent. However, the use of viral vectors in clinic, despite their high transfection activity is limited due to the risk of insertion mutagenesis, an inflammatory response and toxicity. The application of plasmid DNA is a safer method of gene transfer. In a model of musculocutaneous nerve repair with an end-to-end and end-to-side anastomosis an intraoperative administration of a DNA plasmid with *vegf* gene into a distal region resulted in a significantly increased number of myelinated fibers per a unit of the cross-section area of the region distal to the anastomosis site that correlated with a significant increase of the VEGF concentration in Schwann cells [21].

A gene-therapeutic construction can be injected paraneurally. In a sciatic nerve injury model plVEGF was administrated intramuscularly and was combined with hyaluronic acid film sheath which covered the anastomosis site in order to reduce the severity of scarring. The drug intramuscular injection was accompanied with a significant increase of the muscular response amplitude and an increased number of myelinated fibers distal to the anastomosis site against their use as monotherapy [22]. The study performed by Wang F. et al. demonstrated a plVEGF dose-dependent effect when giving the gene therapeutic construction intraneurally after end-to-end suturing of sciatic nerve stumps. The use of a higher dosage resulted in the most pronounced increase of neurophysiological parameters and a less decrease of the calf muscle weight index [23]. Synergism in action of some factors has been found out. For example, a combined use of a VEGF gene-coding plasmid and a plasmid encoding the C-CSF gene in a sciatic nerve injury model demonstrated a more pronounced increase in the number of myelinated fibers and capillaries in the region distal to the end-to-end anastomosis, the maintenance of more neurons in the spinal ganglia as well as the early recovery of the motor function [24]. However, only a part of cells is transfected with plasmid DNA when using gene therapeutic agents in vivo. Consequently, the chance that a cell will be transfected simultaneously with two different gene therapeutic constructions is reduced. Thus, a combination of genetic sequences of two growth factors having a synergistic action in one plasmid is reasonable. The efficacy of this approach has already been demonstrated in an animal model of the spinal cord contusion injury.

During this experiment it has been shown that in a setting of direct injection of 40 µg of a VEGF and FGF2 gene containing plasmid into the spinal cord there was a significant increase of the capillary number in sections made in 1.5 cm of the trauma core. Also based on the behavior test data the recovery of the motor function significantly improved as compared to the control group of animals which were not given the VEGF and FGF2 genes- containing plasmid. Based on the results obtained it was concluded that the application of the double cassette plasmid improves the spinal cord vascularization and reduces the area of destruction of the spinal gray and white matter [25]. However, these results give no idea whether the use of the double-cassette plasmid with VEGF and FGF2 genes is effective in peripheral nerve injuries. This is due to the fact that the mechanism of a contusion injury significantly differs by pathogenesis and a severity degree from a trauma accompanied with neurotmesis which is more specific for peripheral nerves and prevails in the total structure of their injuries. But the most important difference is the different regenerative potential of the spinal cord and peripheral nerves. Thus, it is necessary to determine whether plasmids with growth factor genes can be used for the effective improvement of peripheral nerve regeneration.

### List of Drawings

Figure 1. The repeated approach. The entire sciatic nerve with an autologous graft is visualized.
Figure 2. A view of the upper extremity prior to surgery. Post-traumatic and post-operative indented irregular scars are seen on the anterior and posterolateral surface of the lower, middle and upper third of the right upper arm.
Figure 3. This picture shows the lack of active movements in middle phalanges of the fingers 2-5.
Figure 4. The impaired prehension by all fingers can be seen.
Figure 5. A high-grade atrophy of the hand muscle within the zone innervated by the median and ulnar nerves and the ability to oppose finger 1 to finger 2 only can be seen.
Figure 6. The picture shows the injection of the recombinant plasmid pBud (Kan)-coVEGF-coFGF2 into a repaired nerve (see the text)
Figure 7. The picture shows the application of fibrin glue to prevent the recombinant plasmid leakage.
Figure 8. The picture shows atrophy of the hand and forearm muscles. c) nail changes: hypoplastic;
   d) secretory function (sweating): decreased.
Figure 9. Hook grasp (a bag handle).
Figure 10. Fist grasp.
Figure 11. Tip prehension (finger I-III).
Figure 12. Tip prehension (finger I-III).
Figure 13. Tip prehension (finger I-IV).
Figure 14. A diagram of electromyography results for the thenar muscle group are shown.
Figure 15. Electromyography findings for the hypothenar muscle group.

### The Detailed Description of the Invention

The scope of invention is medicine, its preferable field of use is neurosurgery, traumatology and maxillofacial surgery, treatment of peripheral nerve injuries.

The objective of this invention is to improve the results of reconstructive treatment with the use of a gene-therapeutic construction. A genetic sequence of VEGF and FGF-2 genes is the main component of a double-cassette optimized recombinant plasmid pBud(Kan)-coVEGF-coFGF2.

The substance of the invention achieves the claimed technical result through restoring the motor and sensitivity functions of a damaged nerve with significant reduction of a treatment period including later post-injury periods by means of using the drug composed by a codon-optimized recombinant plasmid pBud(Kan)-coVEGF-coFGF2, presented on SEQ No. 1 in order to induce regeneration of a peripheral nerve in a patient. The closest analogue is Patent RU 3459630 C1 "Stimulation Technique for Neuroregeneration with Genetic Constructions", which describes a method of post-traumatic regeneration of a rat spinal cord when injecting a double-cassette plasmid pBud(Kan)-VEGF-FGF2. The invention is defined by the claims. This invention it to protect the following objects.

The codon-optimized recombinant plasmid pBud(Kan)-coVEGF-coFGF2, containing the VEGF and FGF2-coding genes, presented by a nucleotide sequence SEQ No.1, for use in peripheral nerve regeneration in a patient as set forth in claim 1; for use according to claim 1 in peripheral nerve regeneration by intra-, peri and paraneural administration of the codon-optimized recombinant plasmid pBud(Kan) -coVEGF-coFGF2, presented by the sequence SEQ No.1, in intraoperative or post-operative periods. for use according to claim 1 for treating a damaged human nerve by injecting an effective amount of the plasmid pBud(Kan)-coVEGF-coFGF2 into the area of damage Based on the experience of gene therapeutic agents developing our research group aimed to create an effective product to treat patients with peripheral nerve injuries. For this purpose we have developed various gene therapeutic constructions which differ from each other by the number of encoded transgenes, the transgenes as well as by nucleotide sequences of the same transgenes.

The experience of using gene therapeutic constructions to improve peripheral nerve recovery has already been described [26]. When evaluating the efficacy of using the plasmid simultaneously coding VEGF and FGF2 genes in a diastatic peripheral nerve injury model the drug was injected directly into a distal and proximal ends as well as into an autologous nerve graft equally in a total dose of 45 µg. Rats were used as experimental animals; they were divided into three groups, namely an intact group, a test group where a gene therapeutic construction was administered, and a control group where a phosphate-buffered saline (PBS) solution was injected instead of the study drug.

The evaluation criteria of regeneration dynamics of the peripheral nerve included the neurophysiological parameters such as the nerve conduction velocity and the muscle response amplitude as well as the histological examination findings such as the number of myelinated fibers and the capillary network density. On day 56 following the injection of the plasmid construction, the neurophysiological parameters in the test group were superior to those in the control one, however, they were significantly inferior to those in the intact animals. Based on the histological examination findings, the myelinated fiber number per a unit of the cross-section area was significantly higher in the experimental group as compared to the control one, however, despite this, no effective recovery of the extremity function was observed.

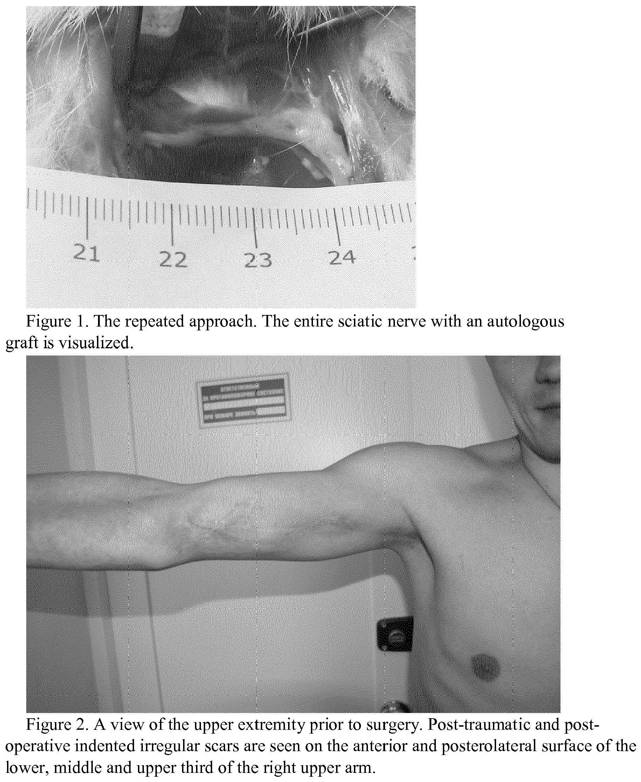

The experiment results have shown that the use of plasmid-based constructions containing genetic sequences of growth factors exert a stimulating effect on the regeneration of peripheral nerves, nevertheless, the effect intensity is suboptimal. We believe that this is primary due to drawbacks of the plasmid itself that was used in our study. In order to improve the properties of the gene-therapeutic construction a number of important changes of its structure were made. At first, the vector was modified: tag sequences were removed and a kanamycine resistance gene was replaced. Secondly, to increase the expression efficacy codon-optimized cDNA sequences of VEGF and FGF2 genes (SEQ No.1) were used. Thereafter we have again conducted a number of experimental studies in a peripheral nerve injury model with the use of the codon-optimized plasmid. Gene therapeutic constructions were administrated intraneurally immediately after the peripheral nerve suturing. The results were evaluated in 60 days following the surgical intervention and drug administration (Fig. 1).

Of all the plasmid DNAs we used the best results were obtained for the codon-optimized double-cassette plasmid containing genetic sequences of FGF-2 and VEGF.

Based on non-clinical data on the efficacy of gene therapeutic constructions pBud(Kan)-VEGF-FGF2 used to improve the peripheral nerve regeneration, we have initiated a clinical study which results are presented below.

Patient B., born in 1985, was admitted to the trauma center of the Republic Clinical Hospital of MoH of the Republic of Tatarstan on April 04, 2011 with the diagnosis of: sequelae of the median and ulnar nerve injury in the middle third of the right upper arm (Fig. 2).

From the history: In 2009 the patient had a glass cut on the middle third of the upper arm, with the median and ulnar nerves damaged. Immediately the median and ulnar nerves were sutured end-to-end, however, both motor and sensitivity functions were completed absent in the immediate post-operative period. A course of rehabilitation therapy produced no visible results. Later on, in 7 months, in 2010 neurolysis of the median and ulnar nerves was performed due to the lack of positive changes in the motor and sensitivity functional recovery. Slight changes in regeneration were observed in the post-operative follow-up, namely, the full lack of sensitivity, at the same time, the motor function appeared which was characterized by mild bending of the hand and fingers, therefore it was decided to carry out a surgical treatment.

Prior to an operation, on April 21, 2011, the patient had an examination done with the results as follows:

### Trophic disturbances:

a) the skin status: of normal color, the decreased fingers' temperature, an increased feeling of chillness;
b) atrophy of the hand and forearm muscles as compared to the normal arm; more than 2 cm (Fig. 2-3);
c) nail changes: hypoplastic;
d) secretory function (sweating): decreased.

### Sensitivity testing in the patient in the autonomous zone of innervation by the nerve:

| No. | Kinds of Sensitivity | Brief Description |
|---|---|---|
| 1. | Pain | Absent |
| 2. | Temperature | Absent |
| 3. | Tactile | Absent |
| 4. | Discriminative | Absent |
| 5. | Sense of two-dimension space | Absent |
| 6. | Stereognosis | Absent |
| 7. | Sense of pressure | Absent |
| 8. | Sense of weight | Absent |

| Degree | Sensitivity recovery |
|---|---|
| S0 | Lack of sensitivity within the nerve autonomous zone |

### Motor function testing

| Degree | Motor function recovery |
|---|---|
| M2 | Distinct contractions without movements in joints |

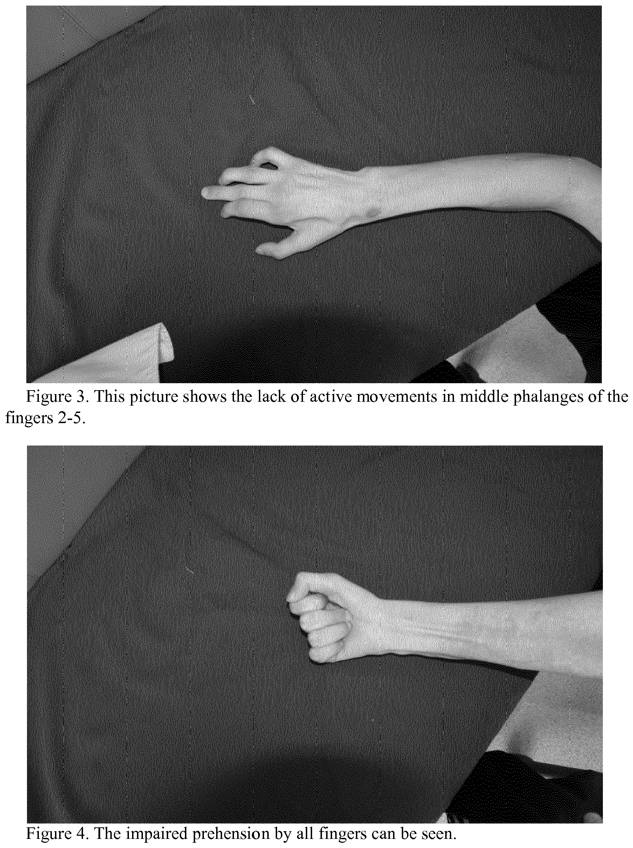

Hand prehension patterns; the hand is unable to perform any type of prehension (Fig. 3-4).

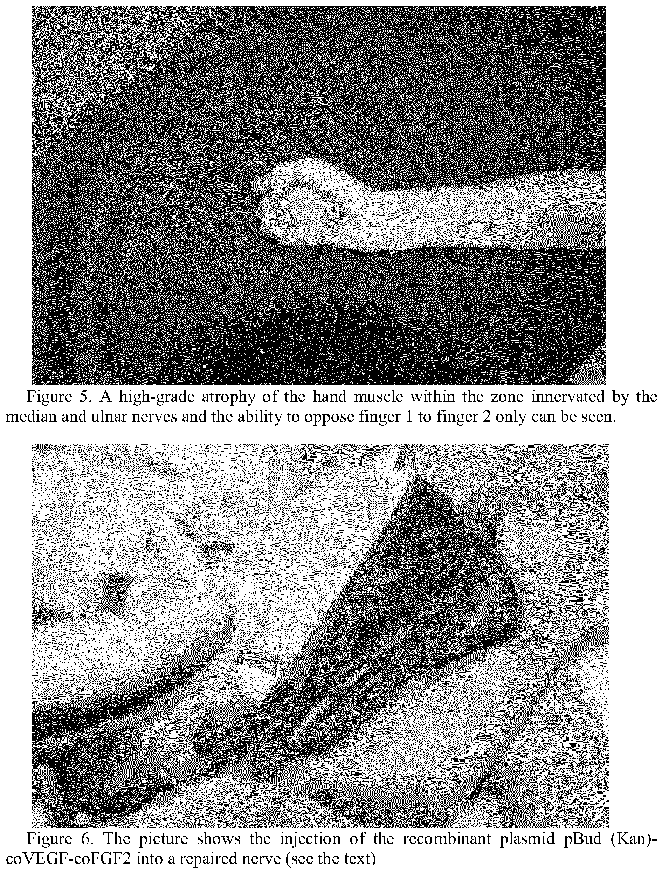

Diagnosis: The injury of the median and ulnar nerves in the middle third of the forearm sustained 2 years ago. The status post suturing and neurolysis of the median and ulnar nerves (Fig. 5).

The operation was performed on April 26, 2011 including neurolysis of the median and ulnar nerves with intraneural administration of the plasmid DNA encodingvegf and *fgf-2* genes.

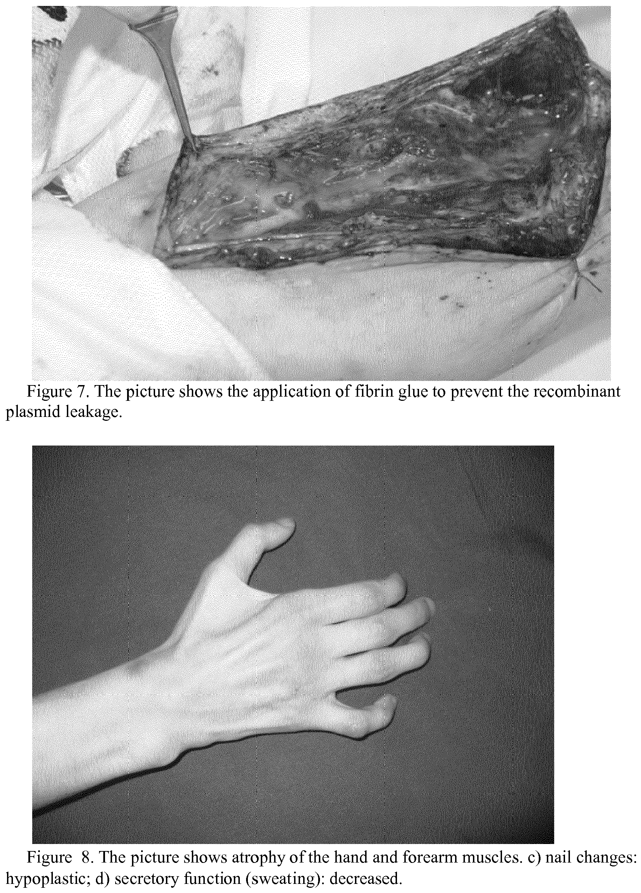

The operation run: Under nerve block anaesthesia, following triple treatment of the surgical field an arcuate incision was made on the inner surface of the right upper arm. The median and ulnar nerves were isolated with a technical difficulty. The suture lines were found out. No neuroma signs observed, however, the nerves are involved in a scar-forming process and adhered to a surrounding tissue. The recombinant VEGF and FGF-2- containing plasmid was injected with an insulin needle, 250 µg per a nerve in 2.5 ml of a physiologic saline solution. The injection was given into a suture zone as well as proximally and distally for 10 cm (Fig. 6). After that 2 ml of a two-component fibrin glue "Tissucol" was applied on the nerves isolated (Fig. 7). Hemostasis. Wound suturing. Rubber tube drainage is placed. Aseptic dressing is applied. A plaster cast is applied. A re-examination was performed in a month after the operation.

The results of physical examination dated on May 25, 2011: Trophic disturbances:
a) the skin status: of normal color;
b) atrophy of the hand and forearm muscles as compared to the normal arm - more than 2 cm (Fig. 8);
c) nail changes: hypoplastic;
d) secretory function (sweating): decreased.

Sensitivity testing in the patient in the autonomous zone of innervation by the nerve:

| No. | Kinds of Sensitivity | Brief Description |
|---|---|---|
| 1. | Pain | Absent |
| 2. | Temperature | Hot - absent |
| | | Cold - distal phalanges of fingers 1,2 |
| 3. | Tactile | Fingers 1,2 - distal phalange |
| 4. | Discriminative | Absent |
| 5. | Sense of two-dimension space (Moberg pickup test) | Absent |
| 6. | Sense of pressure | Present |
| 7. | Sense of weight | present |

| Degree | Sensitivity recovery |
|---|---|
| S1 | Recovery of deep pain sensitivity within the nerve autonomous zone |

### Motor function testing

| Degree | Motor function recovery |
|---|---|
| M2 | Distinct contractions without movements in joints |

Hand prehension patterns: the hand is unable to perform any type of prehension.

A regular examination was performed in 6 months after the operation. The results of physical examination dated on November 15, 2012:

### Trophic disturbances:

a) the skin status: of normal color;
b) atrophy of the hand and forearm muscles as compared to the normal arm - moderate (1-2 cm) and severe - more than 2 cm;
c) nail changes: within normal limits;
d) secretory function (sweating): normal.

Sensitivity testing in the patient in the autonomous zone of innervation by the nerve:

| No. | Kinds of Sensitivity | Brief Description |
|---|---|---|
| 1. | Pain | Present, including distal phalanges of all fingers |
| 2. | Temperature | Hot - distal phalanges of finger 1, middle phalanges of fingers 3, 4 |
| | | Cold - distal phalanges of fingers 1,3; distal phalanges of fingers 2, 4,5 |
| 3. | Tactile | Fingers 1,3 - distal phalange, middle phalange 2, 4,5 |
| 4. | Discriminative | finger 1 - 10 mm |
| | | finger 2 - 30 mm |
| | | finger 3 - 20 mm |
| | | finger 4 - 30 mm |
| | | finger 5 - 30 mm |
| 5. | Sense of two-dimension space (Moberg pickup test) | Identifies large objects (a box of cigarettes, glue, tube for blood collection), a pencil, glue tube |
| 6. | Sense of pressure | Present |
| 7. | Sense of weight | present |

| Degree | Sensitivity recovery |
|---|---|
| S3 | Recovery of surface pain and tactile sensitivity within the entire autonomous zone with complete hyperpathia disappearance |

### Motor function testing

| Degree | Motor function recovery |
|---|---|
| M3 | Mild movements in joints (useful recovery) |

### Hand prehension patterns:

1) cylindrical grasp - YES
2) spherical grasp - YES
3) hook grasp (a bag handle) - YES
4) fist grasp - YES
5) tip prehension
   a) terminal opposition - YES
   b) (subterminal opposition - NO)
6) lateral prehension
   a) pinch grip - NO,
   b) (scissor grip - "cigarette") - NO.

In a year after the operation the patient had a regular examination.

The results of physical examination dated on April 20, 2012:

### Trophic disturbances:

a) the skin status: of normal color;
b) atrophy of the hand and forearm muscles as compared to the normal arm - moderate, 1-2 cm;
c) nail changes: within normal limits;
d) secretory function: within normal limits.

Sensitivity testing in the patient in the autonomous zone of innervation by the nerve:

| No. | Kinds of Sensitivity | Brief Description |
|---|---|---|
| 1. | Pain | Present, including distal phalanges of all fingers |
| 2. | Temperature | Hot - distal phalanges of fingers 1 and 3, middle phalanges of fingers 4, 5 Cold - distal phalanges of fingers 1,3; distal phalanges of fingers 1, 2, 3, 4,5 |
| 3. | Tactile | Fingers 1, 2, 3, 4,5 - distal phalange |
| 4. | Discriminative | finger 1 - 5 mm finger 2 - 5 mm finger 3 - 10 mm finger 4 - 5 mm finger 5 - 10 mm |
| 5. | Sense of two-dimension space (Moberg pickup test) | Identifies large objects (a box of cigarettes, glue, tube for blood collection), as well as small objects (rubber, button, coin, clip) |
| 6. | Sense of pressure | Present |
| 7. | Sense of weight | present |

| Degree | Sensitivity recovery |
|---|---|
| S3+ | Recovery of surface pain and tactile sensitivity within the entire autonomous zone with complete hyperpathia disappearance, but with some recovery of two-point discrimination within the autonomous zone (from 12 to 15 mm) |

### Motor function testing

| Degree | Motor function recovery |
|---|---|
| M4 | Movements with overcoming some resistance |

### Hand prehension patterns:

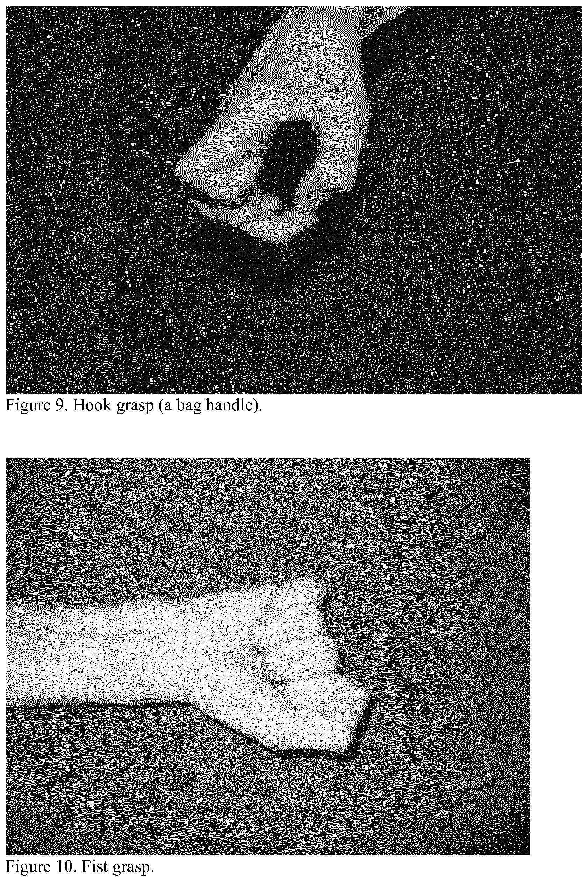

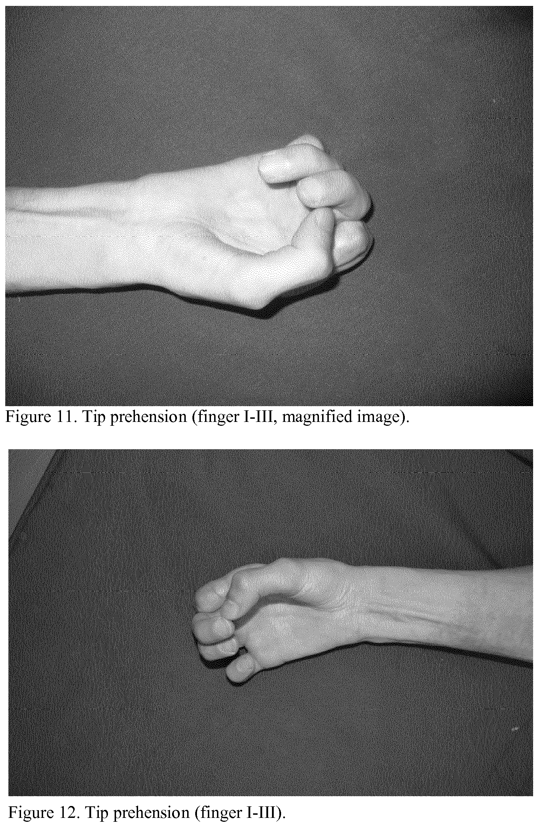

1) spherical grasp - YES
2) spherical grasp - YES
3) hook grasp - YES (Fig. 9)
4) fist grasp - YES (Fig. 10)
5) tip prehension: (Fig. 11-13)
   a) terminal opposition - YES
   b) subterminal opposition - YES
6) lateral prehension
   a) pinch grip - YES
   b) scissor grip - YES.

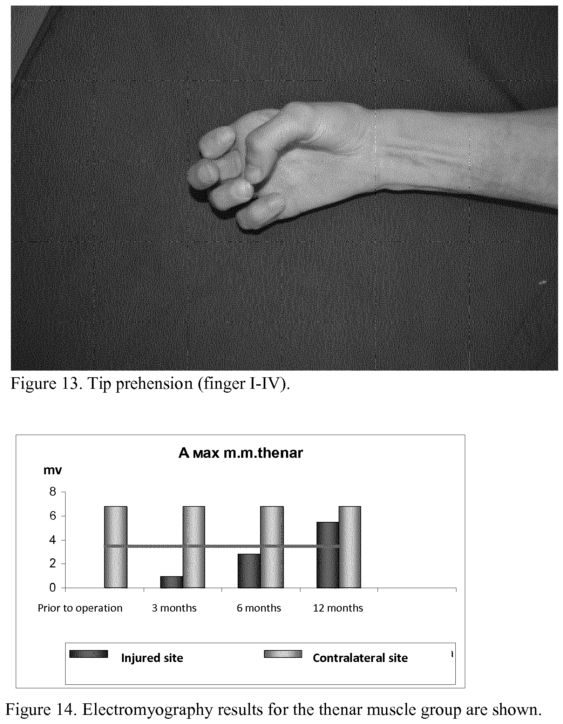

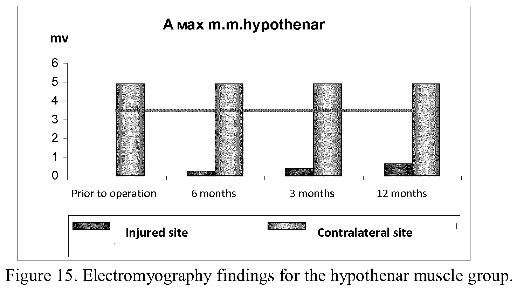

Thus, the clinical study results have shown that the extremity function significantly improved in a year after the intraneural administration of the gene-therapeutic construction. The improved functional state of the extremity manifested as a decreased severity of the trophic disturbances, the development of all kinds of sensitivity within the area of innervation of the median and ulnar nerves, as well as a significant improvement of the motor function. Based on the electromyography results the thenar muscle response amplitude increased over the year from 0 mV to 5 mV and almost achieved the value of the contralateral extremity (Fig. 14 and 15).

The efficacy of the double-cassette plasmid use is due to the feasibility of a simultaneous transfer of two genes, VEGF and FGF2, that promotes a more effective induction of peripheral nerve regeneration. The changes introduced into the codon-optimized plasmid structure enable increasing the expression of the encoded gene significantly, ensuring the required therapeutic concentration of these growth factors in a trauma core that enhance the efficacy of a peripheral nerve recovery. Thus, we suppose that the clinical effect achieved when using the plasmid pBud (Kan)-coVEGF-coFGF2 was obtained due to the combination of these two growth factors and the optimization of their codon sequence.

Unfortunately, at the present stage we are unable to determine definitely the mechanism of induction of peripheral nerve regeneration by means of the above described gene therapeutic construction, that requires further studies. However, the efficacy of their use to improve surgical treatment results of peripheral nerve injuries has been determined and demonstrated in an experiment and a clinical observation.

### References

1. Hudso, A.R. Timing of peripheral nerve repair: important local and neuropathological factors / A.R. Hudson // Clinical Neurosurgery. - 1977. - Vol. 24. - C. 391-405.
2. Deitch, E.A. Experience with 112 shotgun wounds of the extremities/ E.A. Deitch, W.R. Grimes // J Trauma. - 1984. - Vol. 24. - P. 600-603.
3. Analysis of upper and lower extremity peripheral nerve injuries in a population of patients with multiple injuries / C.A. Munro // J. Trauma. - 1998. - Vol. 45. - P. 116-122.
4. Terenghi, G. Peripheral nerve regeneration and neurotrophic factors / G. Terenghi // J. Anat. 1999. - Vol. 194. - P.
5. Vascular endothelial growth factor has neurotrophic activity and stimulates axonal outgrowth, enhancing cell survival and Schwann cell proliferation in the peripheral nervous system / M. Sondell, M. Kanje. G. Lundborg // J Neurosci. - 1999. - Vol. 19, Nº14 - P. 5731-40.
6. VEGF-A165b is an endogenous neuroprotective splice isoform of vascular endothelial growth factor A in vivo and in vitro / N. Beazley-Long [et al.] // J Pathol. - 2013. - Vol. 183, Nº3 - P. 918- 29.
7. Sondell, M. Vascular endothelial growth factor stimulates Schwann cell invasion and neovascularisation of acelular nerve grafts / M. Sondell, G. Lundborg, M. Kanje // Brain Res. -1999. - Vol. 846 - P. 219-228.
8. Vascular, glial and neuronal effects of vascular endothelial growth factor in mesencephalic expiants cultures / W.F. Silverman [et al.] // Neuroscience. - 1999. - Vol. 90 - P. 1529-1541.
9. Forstreuter, F. Vascular endothelial growth factor induces chemotaxis and proliferation of microglial cells / F. Forstreuter, R. Lucius, R. Mentlein // J. Neuroimmunol. - 2002. - Vol. 132 - P. 93-98.
10. Zhu, Y. Vascular endothelial growth factor promotes proliferation of cortical neuron precursors by regulating E2F expression / Y. Zhu [et al.] // J FASEB. - 2003. Vol. 17 - P. 186-193.
11. Induction of VEGF and its Flt-1 receptor after sciatic nerve crush injury / R.R. Islamov [et al.] // Neuroreport. - 2004. Vol. 15, Nº13 - P. 2117-21.
12. Effects of vascular endothelial growth factor on nerve regeneration in acellular nerve grafts / J.M. Rovak [et al.] // J Reconstr Microsurg. - 2004. Vol. 20, Nº1 - P. 53-58.
13. Vascular endothelial growth factor-loaded poly (lactic-co-glycolic acid) microspheres-induced lateral axonal sprouting into the vein graft bridging two healthy nerves: nerve graft prefabrication using controlled release system / H. Karagoz [et al.] // J Microsurgery. - 2012. Vol. 32, Nº8 - P. 635-41.
14. Sondell, M. Vascular endothelial growth factor stimulates Schwann cell invasion and neovascularization of acellular nerve grafts // M. Sondell, G. Lundborg, M. Kanje // Brain Res. -1999. Vol. 846, Nº2 - P. 219-28.
15. The effect of vascular endothelial growth factor and brain-derived neurotrophic factor on cavernosal nerve regeneration in a nerve-crush rat model / PS. Hsieh [et al.] // BJU Int. - 2003. Vol. 92, Nº4 - P. 470-5.
16. Grothe, C. Physiological function and putative therapeutic impact of the FGF-2 system in peripheral nerve regeneration-lessons from in vivo studies in mice and rats / K. Haastert, J. Jungnickel, C. Grothe // Brain Res Rev. - 2006. Vol. 51 - P. 293-299.
17. Furushol, M. Disruption of Fibroblast growth factor receptor signaling in non-myelinating Schwann cells causes sensory axonal neuropathy and impairment of thermal pain sensitivity / M. Furushol [et al.] // J Neurosci. - 2009. Vol. 29, Nº6 - P. 1608-1614.
18. Tulio, V.R. Bone marrow-derived fibroblast growth factor-2 induces glial cell proliferation in the regenerating peripheral nervous system / V.R. Tulio [et al.] // Molecular Neurodegeneration. - 2012. Vol. 7, Nº34 - P. 1-17.
19. Sciatic nerve grafting and inoculation of FGF-2 promotes improvement of motor behavior and fiber regrowth in rats with spinal cord transaction / F.P. Guzen, [et al.] // Restorative Neurology and Neuroscience. - 2012. Vol. 30 - P. 265-275.
20. Zeng, W. Ionically cross-linked chitosan microspheres for controlled release of bioactive nerve growth factor / W. Zeng [et al.] // Int J Pharm. - 2011. Vol. 421 - P. 283-290.
21. Enhancement of musculocutaneous nerve reinnervation after vascular endothelial growthfactor (VEGF) gene therapy / P. Haninec [et al.] // BMC Neuroscience. - 2012. Vol. 13, Nº 57 - P.
22. Effect of VEGF gene therapy and hyaluronic acid film sheath on peripheral nerve regeneration / F. Zor [et al.] // - 2014. Vol. 34, Nº3 - P. 209-16.
23. Favorable effect of local VEGF gene injection on axonal regeneration in the rat sciatic nerve / C. Fu [et al.] // J Huazhong University Scince Technology. - 2007. Vol. 2 - P. 186-9.
24. Double gene therapy with granulocyte colony-stimulating factor and vascular endothelial growth factor acts synergistically to improve nerve regeneration and functional outcome after sciatic nerve injury in mice / F. Pereira Lopes [et al.] // Neuroscience. - 2013. Vol. 230 - P. 184- 97.
25. Pat. 2459630 RF, IPC A61K 48/00, A61P 25/28, C12N 15/79, C1. Stimulation Technique for Neuroregeneration with Genetic Constructions / Yu.A. Chelyshev; Federal State Educational Institution "Kazan Federal University". - Nº2011116853/10; 27.04.2011; published 27.10.2009, Bull. Nº30. - 11 p. [in Russian]
26. Stimulation of post-traumatic regeneration of a rat sciatic nerve with a plasmid expressing the vascular endothelial growth factor and the basic fibroblast growth factor. /R.F. Masgutov [et al]//Cell Technologies and Tissue Engineering. -2011.- 6(3): 67-70. [in Russian].

## Claims

1. Codon-optimized recombinant plasmid pBud(Kan)-coVEGF-coFGF2, containing the VEGF and FGF2-coding genes, presented by a nucleotide sequence SEQ No.1, for use in peripheral nerve regeneration in a patient.

2. The codon-optimized recombinant plasmid pBud(Kan)-coVEGF-coFGF2 for use according to claim 1, wherein said recombinant plasmid is provided for intra-, peri and paraneural administration in intraoperative or post-operative periods.

3. The codon-optimized recombinant plasmid pBud(Kan)-coVEGF-coFGF2 for use according to claim 1, wherein said recombinant plasmid is provided as an injection into the area of damage for the treatment of a damaged human nerve.

## Patentansprüche

1. Codon-optimierter rekombinanter Plasmid pBud(Kan)-coVEGF-coFGF2, enthaltend die VEGF- und FGF2-kodierenden Gene, dargestellt durch eine Nukleotidsequenz SEQ Nr. 1, zur Verwendung bei der peripheren Nervenregeneration bei einem Patienten.

2. Codon-optimierter rekombinanter Plasmid pBud(Kan)-coVEGF-coFGF2 zur Verwendung nach Anspruch 1, wobei der rekombinante Plasmid zur intra-, peri- und paraneuralen Verabreichung in intraoperativen oder postoperativen Perioden bereitgestellt wird.

3. Codon-optimierter rekombinanter Plasmid pBud(Kan)-coVEGF-coFGF2 zur Verwendung nach Anspruch 1, wobei besagter rekombinanter Plasmid als Injektion in den Schädigungsbereich zur Behandlung eines geschädigten menschlichen Nervs bereitgestellt wird.

## Revendications

1. Plasmide recombinant à codons optimisés pBud(Kan)-coVEGF-coFGF2, contenant les gènes codant pour VEGF et FGF2, présentés par une séquence nucléotidique SEQ No. 1, pour utilisation dans la régénération de nerf périphérique chez un patient.

2. Plasmide recombinant à codons optimisés pBud(Kan)-coVEGF-coFGF2 pour utilisation selon la revendication 1, ledit plasmide recombinant étant fourni pour administration infra-, péri- et paraneuronale dans des périodes peropératoires ou postopératoires.

3. Plasmide recombinant à codons optimisés pBud(Kan)-coVEGF-coFGF2 pour utilisation selon la revendication 1, ledit plasmide recombinant étant fourni sous forme d'injection dans la zone de dommage pour le traitement d'un nerf humain endommagé.
